(19) Europäisches Patentamt
European Patent Office
Office européen des brevets

(11) **EP 2 471 899 A1**

(12) **EUROPEAN PATENT APPLICATION**
published in accordance with Art. 153(4) EPC

(43) Date of publication:
**04.07.2012 Bulletin 2012/27**

(51) Int Cl.:
***C12M 3/00*** *(2006.01)* ***C12N 5/071*** *(2010.01)*

(21) Application number: **10812012.2**

(22) Date of filing: **27.08.2010**

(86) International application number:
**PCT/JP2010/064627**

(87) International publication number:
**WO 2011/024963 (03.03.2011 Gazette 2011/09)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **27.08.2009 JP 2009216200**

(71) Applicant: **Tokyo Women's Medical University Tokyo 162-8666 (JP)**

(72) Inventors:
• **TAKAHASHI, Hironobu**
 **Tokyo 162-8666 (JP)**

• **NAKAYAMA, Masamichi**
 **Tokyo 162-8666 (JP)**
• **OKANO, Teruo**
 **Tokyo 162-8666 (JP)**

(74) Representative: **HOFFMANN EITLE**
 **Patent- und Rechtsanwälte**
 **Arabellastraße 4**
 **81925 München (DE)**

(54) **TEMPERATURE-RESPONSIVE CELL CULTURE SUBSTRATE ON WHICH A STRAIGHT-CHAIN TEMPERATURE-RESPONSIVE POLYMER IS IMMOBILIZED, AND MANUFACTURING METHOD THEREFOR**

(57) Provided is a temperature-responsive cell culture substrate. A non-crosslinked temperature-responsive polymer having a molecular weight between 10,000 and 150,000 is immobilized on the substrate surface with a density of 0.02 to 0.3 molecular chain per square nanometer. Using the provided temperature-responsive cell culture substrate, cells obtained from various tissues can be efficiently cultured. This culturing method makes it possible to efficiently peel off a cell sheet by just changing the temperature, without causing damage.

**Description**

TECHNICAL FIELD

The present invention relates to a cell culture substrate useful in fields including drug development, pharmacy, medicine and biology and a manufacturing method therefor.

BACKGROUND ART

The recent advance in the animal cell culturing technology is significant, and research and development of animal cells are conducted in a wider range of fields. Animal cells, constituting a research object, are now used not only in commercializing the original state of the developed cells or in commercializing the product of such cells, but also in designing effective pharmaceutical products based on the analysis of the cells or the cell cortex protein thereof, and in regenerating a patient's cell or increasing the cell's functions *in vitro,* then returning the cell *in vivo* to treat a patient. Researchers are now focusing on this field of the animal cell culturing technology and the technology for assessing, analyzing and using the former technology.

Various animal cells including human cells are attachment-dependent. In other words, animal cells need to be attached to an object when the cells are cultured *in vitro.* Accordingly, many researchers have sought to culture extracted biogenic cells in an environment that is as close to the *in vivo* environment as possible by, for example, designing substrates coated specifically with cell-adhesive proteins, such as collagen, fibronectin and laminin, and making inventions related to such substrates. However, all such techniques relate to the culturing period of the cells. Adhesion-dependency culture cells produce their own adhesive proteins when they attach to an object. The detachment of such cells requires the destruction of adhesive proteins in the conventional art. The detachment is normally achieved by enzyme processing. An essential problem of such processing is that it concurrently destroys cell-specific cell cortex proteins, which were 9produced by the cells during cell culturing. However, there was no actual means for solving the problem, nor was such means specifically considered. To make significant advances in the research and development of animal cells, the problem concerning cell collection must be solved.

Based on the above background, Patent Document 1 teaches a novel cell culturing method for peeling off cultured cells without enzyme processing by culturing cells on a cell culture support covered with a polymer of a temperature no higher than the upper critical solution temperature or a temperature no lower than the lower critical solution temperature, wherein the upper or lower critical solution temperature of the polymer to water is 0 to 80°C, then changing the polymer temperature to the upper critical solution temperature or higher or to the lower critical solution or lower. Further, Patent Document 2 teaches peeling off cultured dermal cells with little damage by using a temperature-responsive cell culture substrate to culture the dermal cells at a temperature no higher than the upper critical solution temperature or a temperature no lower than a lower critical solution temperature, then changing the substrate temperature to the upper critical solution temperature or higher or the lower critical solution temperature or lower. Furthermore, Patent Document 3 teaches repairing the cortex protein of the cultured cells using such temperature-responsive cell culture substrate. The use of a temperature-responsive cell culture substrate enables various new developments from conventional culturing technology.

Further advances are seen concerning the above temperature-responsive cell culture substrate surfaces. An example taught in Non-Patent Document 1 is a chitosan film having a temperature-responsive polymer grafted on to the chitosan gel film through radical polymerization to provide a more efficient control of cell adhesion and cell detachment based on temperature change. Further, Patent Document 4 presents a surface of a cell culture substrate, wherein a region covered with a temperature-responsive polymer and a cell-adhesive region co-exist on the surface. However, these techniques are mere combinations of a substrate that allows cell adhesion and a temperature-responsive polymer, and they are not substrate surfaces whose cell adhesion, cell proliferation and cell detachment according to temperature change are strictly designed.

A different technical example attempting to strictly design the substrate surface is a cell culture substrate surface having styrene macromonomers spin coated thereon, wherein the styrene macromonomer contains as its component a temperature-responsive polymer whose molecular weight (chain length) is controlled. However, this technique merely optimizes the amount of temperature-responsive polymer to be immobilized on the substrate surface, and does not necessarily provide a surface whose cell adhesion and detachment according to temperature change are strictly designed (Non-Patent Document 2). Further, Patent Document 5 presents a substrate surface whose cell adhesion was improved by the immobilization of cell adhesive factors in a temperature-responsive polymer, but this technique is similarly a mere combination of a substrate that allows cell adhesion and a temperature-responsive polymer, and it is not a substrate surface whose cell adhesion, cell proliferation and cell detachment according to temperature change are strictly designed.

As seen above, the use of a temperature-responsive cell culture substrate enabled various new developments from conventional culture technology. However, the conventional temperature-responsive cell culture substrates were designed for features common to many cells, and their surfaces were not created for more efficient adhesion and proliferation of the cultured cells or for efficient cell detachment based on temperature change alone. Further, the conventional temperature-responsive cell culture substrate was not specially designed according to the features of individual cells collected from different tissues.

Under the above situation, the living radical polymerization is recently receiving attention as a precise method for constructing polymer chains on the substrate surface. This method produces polymers having a significantly narrow molecular-weight distribution compared to the conventional radical polymerization. The Reversible Addition-Fragmentation Chain Transfer Radical Polymerization (RAFT Polymerization) is a technique in which radical species generated from a polymerization initiator induces monomer polymerization via a RAFT agent (Non-Patent Document 3). Accordingly, the technique enables a precise control of the molecular-weight of the resulting polymer by adjusting the concentration ratio of the initiator, the RAFT agent and the monomer. It is expected that a precisely controlled surface can be obtained when the temperature-responsive polymer is immobilized on a surface by a RAFT polymerization reaction initiated from the surface, as in the present invention. That surface will have brush-like temperature-responsive polymers of a uniform molecular-weight immobilized on it. The physical property (temperature-responsiveness) of a surface modified by PIPAAm (poly-N-isopropylacrylamide) depends on the chain length and density of PIPAAm, so the present method leads to a precise control of the temperature-responsiveness of the substrate surface. Also, the polymerization of monomers through a RAFT agent will induce a functional group derived from the RAFT agent to occupy a polymer chain terminal. The terminal functional groups of the above polymers were replaced by various functional groups and reported as terminal-modified polymers in past researches. Such terminal-modified polymer is one advantage of using the RAFT polymerization (Non-Patent Document 4). The above technique is extremely useful in designing a surface for performing cell culture, but no assessment has been made by using cells on a surface created by this technique, which left the possibility of surface design for future studies.

PRIOR ART DOCUMENTS

PATENT DOCUMENTS

Patent Document 1: Japanese Patent Application Publication (Kokai) No. H02-211865
Patent Document 2: Japanese Patent Application Publication (Kokai) No. H05-192138
Patent Document 3: Japanese Patent Application Publication (Kokai) No. 2008-220354
Patent Document 4: Japanese Patent Application Publication (Kokai) No. H08-103653
Patent Document 5: Japanese Patent Application Publication (Kokai) No. H07-135957

NON-PATENT DOCUMENTS

Non-Patent Document 1: Biotechnology and Bioengineering, 101(6), 1321-1331 (2008)
Non-Patent Document 2: Biomaterials 29, 2073-2081 (2008)
Non-Patent Document 3: Aust. J. Chem., 58, 379-410 (2005)
Non-Patent Document 4: Biopolymers, 6, 2320-2327 (2005)

SUMMARY OF THE INVENTION

PROBLEM TO BE SOLVED BY THE INVENTION

The present invention was made with an intent to solve the above problems of the conventional art. That is, the present invention aims to provide a temperature-responsive cell culture substrate that induces efficient adhesion and proliferation of cells useful in fields including medicine, biology and biochemistry, and allows such cultured cells to peel off by temperature change alone, and a manufacturing method therefor.

MEANS TO SOLVE THE PROBLEM

The present inventors performed research and development to solve the above problem by studying the problem from various angles. It was consequently found that the living radical polymerization initiated from an initiator that is immobilized on the substrate surface results in a temperature-responsive cell culture substrate composed of a non-crosslinked temperature-responsive polymer having a molecular weight between 10,000 and 150,000 immobilized on its surface at a density of 0.02 to 0.3 molecular chain/nm$_2$. It was further found that the use of such cell culture substrate allows more efficient adhesion and proliferation of the cells compared to conventional temperature-responsive culture substrate and that the cultured cells could be peeled off by temperature change alone. The technique described in the present invention was not predictable from the conventional art and raises expectation for the development of a novel cell culture substrate which did not exist in the conventional art. The present invention was completed with such insight as its basis.

That is, the present invention provides a temperature-responsive cell culture substrate comprising a substrate surface that a non-crosslinked temperature-responsive polymer having a molecular weight between 10,000 and 150,000 is immobilized on at a density of 0.02 to 0.3 molecular chain/nm$^2$. The present invention further provides a manufacturing method of the temperature-responsive cell culture substrate comprising living radical polymerization as a method for fixing the temperature-responsive polymer to the substrate surface.

ADVANTAGEOUS EFFECT OF THE INVENTION

The temperature-responsive cell culture substrate obtained in the present invention enables efficient culturing of cells collected from different tissues. Further, the use of the culturing method that uses the above temperature-responsive cell culture substrate enables cultured cells to peel off efficiently.

BRIEF DESCRIPTION OF THE DRAWINGS

[Figure 1] Figure 1 is data showing the element compositions of the PIPAAm graft substrate surface and the initiator immobilized substrate surface (V-501 substrate) of Example 1 measured by XPS.
[Figure 2] Figure 2 is a chart showing the FT-IR measurement result of PIPAAm graft substrate surface of Example 1.
[Figure 3] Figure 3 is a micrograph showing the surface-attached cells of Example 1, 6 hours after cell dissemination (Scale bar: 200 $\mu$m).
[Figure 4] Figure 4 is a micrograph showing the cells of Example 1, cultured to a confluent state 5 days after cell dissemination (Scale bar: 100 $\mu$m).
[Figure 5] Figure 5 is a photograph showing a cell sheet of Example 1, after 30 minutes of cooling.
[Figure 6] Figure 6 is a micrograph showing the surface-attached cells of Example 2, 6 hours after cell dissemination (Scale bar: 200 $\mu$m).
[Figure 7] Figure 7 is a micrograph showing the cells of Example 2, cultured to a confluent state 2 days after cell dissemination (Scale bar: 100 $\mu$m).
[Figure 8] Figure 8 is a photograph showing a cell sheet of Example 2, after 30 minutes of cooling.
[Figure 9] Figure 9 is a micrograph showing the cells of Example 3, cultured to a confluent state 2 days after cell dissemination (Scale bar: 100 $\mu$m).
[Figure 10] Figure 10 is a micrograph showing a cell sheet of Example 3, after 1 hour of cooling (Scale bar: 100 $\mu$m).
[Figure 11] Figure 11 is a micrograph showing the cells of Comparative Example 1, cultured to a confluent state 2 days after cell dissemination (Scale bar: 100 $\mu$m).
[Figure 12] Figure 12 is a micrograph showing a cell sheet of Comparative Example 1, after 24 hours of cooling (Scale bar: 100 $\mu$m).
[Figure 13] Figure 13 is a micrograph showing the adhesion behavior (3 hours after dissemination) of RPTEC that was disseminated to the brush surface of the unmodified PIPAAm (Scale bar: 100 $\mu$m).
[Figure 14] Figure 14 is a micrograph showing the adhesion behavior (3 hours after dissemination) of RPTEC that was disseminated to the brush surface of RGD-modified PIPAAm (Scale bar: 100 $\mu$m).
[Figure 15] Figure 15 is a micrograph showing the adhesion behavior (24 hours after dissemination) of RPTEC that was disseminated to the brush surface of the unmodified PIPAAm (Scale bar: 100 $\mu$m).
[Figure 16] Figure 16 is a micrograph showing the adhesion behavior (24 hours after dissemination) of RPTEC that was disseminated to the brush surface of the RGD-modified PIPAAm (Scale bar: 100 $\mu$m).
[Figure 17] Figure 17 is a micrograph showing the desorption behavior of RPTEC in a low temperature processing (culture at 20˚C, after 2 hours) that attached to the brush surface of the RGD-modified PIPAAm (Scale bar: 100 $\mu$m).

BEST MODE FOR CARRYING OUT THE INVENTION

The present invention relates to a cell culture substrate having a temperature-responsive surface with a non-crosslinked temperature-responsive polymer immobilized on it. Specifically, the present invention relates to a temperature-responsive cell culture substrate composed of a non-crosslinked temperature-responsive polymer having a molecular weight between 10,000 and 150,000 immobilized on its surface at a density of 0.02 to 0.3 molecular chain/nm$^2$. The polymer immobilized on the cell culture substrate surface in the present invention is a linear polymer chain. The molecular weight of such polymer should be 10,000 to 150,000, preferably 80,000 to 130,000, and more preferably 100,000 to 120,000. A substrate surface having a molecular weight that is lower than 10,000 is not preferable as a substrate surface of the present invention, because such surface cannot achieve enough hydrophilicity to cause cells to peel off by a temperature change. In contrast, a substrate surface that has a polymer with a molecular weight that is higher than 150,000 is not preferable as a temperature-responsive culture substrate of the present invention, because such surface does not allow cell adhesion at any temperature range that immobilizes the polymer chain on the substrate surface. Further, the present invention is characterized by the above polymer chain immobilized at a density of 0.02 to 0.3 molecular chain/nm2, preferably 0.03 to 0.2 molecular chain/nm$^2$, more preferably 0.04 to 0.1 molecular chain/nm$^2$. A substrate surface having an immobilization density that is lower than 0.02 molecular chain/nm$^2$ is not preferable as a substrate surface of the present invention, because such surface cannot achieve enough hydrophilicity for cells to peel off by temperature change. In contrast, a substrate surface that has a polymer immobilized on it at an excessively high density, which is higher than 0.3 molecular chain/nm$^2$, is not preferable as a temperature-responsive culture substrate of the present invention, because a steric hindrance occurs on the polymer chain during the polymerization process and hinders polymerization, which hinders immobilization of sufficient polymers to exhibit a hydrophilic surface at temperature change, and consequently, prevents cultured cells from peeling off when the temperature changes. The above temperature-responsive polymers immobilized on the substrate surface have narrow molecular weight distribution. That is, temperature-responsive polymers of uniform molecular mass are immobilized on the substrate surface. The molecular weight dispersion of a dispersion ratio, Mw/Mn, is in the range of 1.1 to 1.5 and normally, 1.2 to 1.3.

As described above, the molecular weight of the temperature-responsive polymer immobilized on the cell culture substrate surface and the immobilization density of the polymer chains of such polymer strongly affect the adhesion, proliferation, and detachment of the cells cultured on the substrate surface. Another matter determined by the molecular weight of the temperature-responsive polymer immobilized on the cell culture substrate surface and the immobilization density of the polymer chains of such polymer is the amount of temperature-responsive polymer to be coated on the substrate surface; it's range should be 0.03 to 2.4 $\mu$g/cm$^2$, preferably 0.05 to 1.8 $\mu$g/cm$^2$, and more preferably 0.1 to 1.5 $\mu$g/cm$^2$. An amount of coating that is lower than 0.03 $\mu$g/cm$^2$ is not preferable, because such amount will prevent the cultured cells on the polymer from peeling off when the temperature changes and thus make work efficiency significantly poor. In contrast, an amount higher than 2.4 $\mu$g/cm$^2$ is not preferable for a cell culture substrate of the present invention, because cell adhesion to such region will be difficult, and sufficient cell adhesion will be hindered. The amount of coating can be measured according to conventional methods. Examples of such methods include the FT-IR-ATR method, the elemental analysis method, and the ESCA, and any of these methods can be used. The above description is integrated into a specific example of a temperature-responsive cell substrate surface of the present invention for culturing vascular endothelial cells, which is a substrate surface having a temperature-responsive polymer immobilized thereon at a molecular weight of 110,000, an immobilization density of 0.036 molecular chain/nm$^2$, and in a coating amount of 0.65 $\mu$g/cm$^2$. Similarly, a substrate surface for culturing fibroblast includes that having a temperature-responsive polymer immobilized thereon at a molecular weight of 90,000, an immobilization density of 0.05 molecular chain/nm$^2$, and a coating amount of 0.74 $\mu$g/cm$^2$. Further, a substrate surface for culturing epithelial cells includes that having a temperature-responsive polymer immobilized thereon at a molecular weight of 125,000, an immobilization density of 0.07 molecular chain/nm$^2$, and a coating amount of 1.45 $\mu$g/cm$^2$.

In the present invention, a temperature-responsive polymer that changes hydration at a temperature from 0 to 80°C is immobilized on the substrate surface. The immobilization method is not particularly limited as long as the temperature-responsive polymer is immobilized by the living radical polymerization starting from an initiator that is immobilized on the substrate surface. An example is a method of immobilizing the polymerization initiator on the substrate surface, then bringing about a growth reaction of a temperature-responsive polymer from that initiator under a catalyst using the Atom Transfer Radical Polymerization (ATRP polymerization). The initiator to be used in the process is not particularly limited, but an initiator to be used when the substrate is silica or glass as in the present invention includes 1-trichlorosilyl-2-(m-chloromethylphenyl)ethane, 1-trichlorosilyl-2-(p-chloromethylphenyl)ethane, a mixture of 1-trichlorosilyl-2-(m-chloromethylphenyl)ethane and 1-trichlorosilyl-2-(p-chloromethylphenyl)ethane, 2-(4-chlorosulfonyl-phenyl)ethyltrimethoxysilane and (3-(2-bromoisobutyryl)propyl)dimethylethoxysilane. A polymer chain is grown from such initiator in the present invention. The catalyst used in the process is not particularly limited, but copper halides (Cu$^I$X), such as Cu$^I$Cl or Cu$^I$Br, can be used when N-alkylsubstituted(meth)acrylamide derivates are selected as polymers whose hydration change. Ligand complexes corresponding to the copper halides are not particularly limited either, but such complexes include tris(2-(dimethylamino)ethyl)amine (Me$_6$TREN), N,N,N'',N''-pentamethyldiethylenetriamine (PMDETA), 1,1,4,7,10,10-hexamethyltriethylenetetraamine (HMTETA), 1,4,8,11-tetramethyl-1,4,8,11-azacyclotetradecane (Me$_4$Cyclam), bipyridine. Further, another method is a method for inducing the growth reaction of a temperature-responsive polymer by a surface-initiated radical polymerization, wherein the reaction is started from the initiator immobilized on the above substrate surface, and brought about by the Reversible Addition-Fragmentation Chain Transfer Radical Polymerization (RAFT Polymerization), under the coexistence of a RAFT agent. The initiator used in the process is not particularly limited, but an initiator to be used when the substrate is silica or glass as in the present invention includes 2,2'-azobis(isobutyronitrile), 2,2'-azobis(4-methoxy-2,4-dimethylvaleronitrile) (V-70), 2,2'-azobis[(2-carboxyethyl)-2-(methylpropionamidine)] (V-057) reacted via a silane coupling agent. In the present invention, polymers are grown from the above initiators. The RAFT agent used in the process is not particularly limited, but such RAFT agent includes benzyldithiobenzoate, cumyl dithiobenzoate, 2-cyanopropyldithiobenzoate, 1-phenylethyl phenyldithioacetate, cumyl phenyldithioacetate, benzyl1-pyrrolecarbodithioate, and cumyl1-pyrrolecarbodithioate.

The solvent to be used at polymerization in the present invention is not particularly limited, but isopropylalcohol (IPA) is preferable in the ATRP polymerization. The inventors performed various studies. They first selected N-ispropylacrylamide as the ingredient of the temperature-responsive polymer and performed the Atom Transfer Radical Polymerization at room temperature in a solution, and found that the reaction rate is substantially the same for any reaction solvent selected from dimethylformaldehyde (DMF), water and IMF. They also found in contrast that a solid-phase reaction like the present invention which aims to immobilize and polymerize N-isopropylacrylamide on a solid substrate surface exhibits an extremely low reaction rate when IPA is selected as the reaction solvent instead of the other two. The inventors further found that if t-butylatcohol described in the above Macromolecules 38, 5937-5943 (2005) is selected, it may solidify at room temperature, so a reaction temperature higher than the room temperature is required and the reaction rate will consequently increase; thus, t-butylalcohol is unsuitable for the present invention. Such insight was not known in the conventional art, and the present invention shows that polymerization by immobilization to the carrier exhibits a gradual increase of the molecular weight of the polymer chain, and a gradual increase in the amount of polymer chains immobilized on the carrier surface when IPA is selected as the reaction solvent. Hence, polymer chains can be uniformly immobilized on the carrier surface according to the method of the present invention. Further, the termination of the reaction at a predetermined time enables carriers maintaining the immobilized state of the reaction termination timing to be produced with excellent reproducibility. A preferable solvent to be used in the RAFT polymerization includes 1,4-dioxane, dimethylformaldehyde (DMF). The above solvent is not particularly limited, but it can be selected as necessary according to the types of monomers, RAFT agents and polymerization initiators used in the polymerization reaction.

The present invention immobilizes the temperature-responsive polymer as a coating by a living radical polymerization from the initiator immobilized on the carrier surface. The Atom Transfer Radical Polymerization (ATRP method) is a method for the growth reaction of a charged polymer that changes hydration in a temperature range of 0˚C to 80˚C. The method includes immobilizing an ATRP polymerization initiator and performing the Atomic Transfer Radical Polymerization from the initiator under a polymerization catalyst, wherein the solvent can be isopropylalcohol, as mentioned above. Other conditions, such as the initiator concentration, copper halide concentration, ligand complex concentration, reaction temperature and reaction duration of the polymerization, are not particularly limited, and can be changed according to the purpose. The reaction solution may be kept still or stirred, but the latter state is preferable to achieve a uniform immobilization on to the carrier surface. The Reversible Addition-Fragmentation Chain Transfer Radical Polymerization is a method for the growth reaction of a polymer that changes hydration in a temperature range of 0˚C to 80˚C. The method includes immobilizing a RAFT polymerization initiator and performing surface-initiated radical polymerization from that initiator under the co-existence of a RAFT agent by using solvents such as 1,4-dioxane. Other conditions, such as the RAFT agent concentration, reaction temperature and reaction duration of the polymerization, are not particularly limited, and can be changed according to the purpose. The reaction solution may be kept still or stirred, but the latter state is preferable to achieve a uniform immobilization on to the carrier surface. Further, unlike the ATRP method, the RAFT method requires no metal ion to immobilize the temperature-responsive polymer, and advantageously eliminates the trouble of washing the substrate after immobilizing the temperature-responsive polymer. Further, the polymerization conditions per se are more simple and advantageous in the RAFT method.

The material of the cell culture substrate to be coated is not limited to substances commonly used in cell culture, such as glass, modified glass, polystyrene, polymethylmethacrylate, but all general shapeable substances can be used including polymer compounds other than the above, ceramics, metals. The shape of the substrate is not limited to cell culture dishes, such as a petri dish, but it can include plates, fibers and (porous) particles. It can further be in the shape of a container (flask) commonly used for cell culture or other purposes.

The temperature-responsive polymer used in the present invention has an upper critical solution temperature or a lower critical solution temperature in an aqueous solution of 0˚C to 80˚C, and more preferably 20˚C to 50˚C. An upper critical solution temperature or a lower critical solution temperature that is higher than 80˚C is not preferable, because the cells may die out. An upper critical solution temperature or a lower critical solution temperature that is lower than 0˚C is also not preferable, because the cell proliferation rate decreases extremely or the cells die out. The temperature-responsive polymer used in the present invention may be either a single polymer or a copolymer. Such polymers include the polymer described in Japanese Patent Open Publication No. H02-211865. Such polymers can specifically be obtained, for example, from the polymerization of a single monomer or a copolymerization of the monomers below.

Monomers that can be used include (meth)acrylamide compounds, N-(or N,N-di)alkylsubstituted(meth)acrylamide derivatives, or vinylether derivatives. Two or more types of such monomers can be used in a copolymer. Additionally, a copolymerization with monomers other than the above monomers, a graft or copolymerization among polymers, or a mixture of a single polymer and a copolymer may be used. Polymers can also be cross-linked as long as the intrinsic characteristics of the polymers are not impaired. Temperature-responsive polymers usable in the present invention include the following, in view of the object to be cultured and peeled off being cells, which require separation to be performed at a temperature between 5˚C and 50˚C: poly-N-n-propylacrylamide (lower critical solution temperature of the single polymer: 21˚C), poly-N-n-propylmethacrylamide (the above described temperature: 27˚C), poly-N-isopropylacrylamide (the above described temperature: 32˚C), poly-N-isopropylmethacrylamide (the above temperature: 43˚C), poly-N-cyclopropylacrylamide (the above described temperature: 45˚C), poly-N-ethoxyethylacrylamide (the above described temperature: about 35˚C), poly-N-ethoxyethylmethacrylamide (the above described temperature: about 45˚C), poly-N-tetrahydrofurfurylacrylamide (the above described temperature: about 28˚C), poly-N-tetrahydrofurfurylmethacrylamide (the above described temperature: about 35˚C), poly-N,N-ethylmethylacrylamide (the above described temperature: 56˚C), poly-N,N-diethylacrylamide (the above described temperature: 32˚C). Monomers to be used in the copolymerization of the present invention include without limitation acrylamide, N,N-diethylacrylamide, N,N-dimethylacrylamide, ethylene oxide, acrylic acid and a salt thereof, hydroxyethylmethacrylate, hydroxyethylacrylate, vinylalcohol, and vinylpyrrolidon, and polymers to be used include without limitation hydrated forms of polymers, such as polyacrylamide, poly-N,N-diethylacrylamide, poly-N,N-dimethylacrylamide, polyethylene oxide, polyacrylic acid and a salt thereof, polyhydroxyethylmethacrylate, polyhydroxyethylacrylate, polyvinylalcohol, polyvinylpyrrolidon, cellulose, and carboxymethylcellulose.

When a surface-initiated radical polymerization using a RAFT agent is used in the present invention, the resulting polymer will have a dithio ester functional group, which is a part of the RAFT agent structure, remaining on its terminal. This phenomenon is characteristic of the RAFT polymerization and allows a new polymerization reaction to start from a terminal of a polymer after the polymerization reaction producing that polymer has completed. As a result of using such polymer, a surface composed of a block copolymer, which differs from conventional copolymers, is produced. Adding a compound, such as 2-ethanolamine, when the new polymerization reaction starts will allow a dithio ester functional group on the terminal of the temperature-responsive polymer to easily be substituted with a thiol group. Such reaction requires no special condition, is simple, and progresses in a short time. Such reaction results in a polymer chain having a highly reactive thiol group, and allows terminals of the obtained polymer chain to be selectively and efficiently modified by a functional molecule having a function group, such as a maleimide group or a thiol group. Hence, new functionalities can be added to the surface of the temperature-responsive culture substrate. Function groups to be added are not particularly limited, but such function groups include a hydroxyl group, a carboxyl group, an amino group, a carbonyl group, an aldehyde group, a sulfonic acid group. In addition, peptides and proteins, which stimulate cell adhesion, can be immobilized on the terminal of the polymer chain. In view of the fact that the lower critical solution temperature (LCST) of poly-N-isopropylacrylamidc varies according to the hydrophilicity and/or hydrophobicity of the terminal functional group, introducing a functional group to a polymer chain terminal in the present invention is expected to provide a new method for controlling the temperature-responsiveness of the substrate surface, from an unconventional view point.

The cells to be used against the surface of the temperature-responsive cell culture substrate obtained in the present invention are not limited as long as they are animal cells, nor are the source and manufacture method thereof particularly limited. Cells to be used in the present invention include cells of animals, insects or cells of bacteria. Specifically, the sources of animal cells include without particular limitation, humans, monkeys, dogs, cats, rabbits, rats, nude mice, mice, guinea pigs, pigs, sheep, Chinese hamsters, cows, marmosets, African green monkeys. Further, the culture medium to be used in the present invention is not particularly limited as long as it is a medium for culturing animal cells, but examples of such medium include serum-free culture medium and serum-containing culture medium. Differentiation inducing substances including a retinoic acid and an ascorbic acid can be further added to the above culture medium. The dissemination density on the substrate surface is not particularly limited as long as the dissemination is performed according to the conventional method.

Further, the temperature-responsive cell culture substrate of the present invention enables cultured cells to peel off without enzyme processing by the temperature of the culture substrate being adjusted to the upper critical solution temperature or higher or the lower critical solution temperature or lower of the polymer coating on the cell substrate. The process can be performed in a culture solution or other isotonic solutions, and the solution can be selected according to the purpose. Methods, such as lightly tapping or swaying the substrate, or further, stirring the culture medium using a pipet, can be used alone or in combination to peel off and collect the cells more quickly and efficiently.

The use of a temperature-responsive cell culture substrate according to the present invention enables efficient culturing of cells obtained from different tissues. The use of this culture method allows cells to peel off efficiently without damage by temperature change alone. Such operation conventionally required effort and skill of the person performing the operation, but the present invention eliminates such requirements and enables mass processing of cells. The present invention shows that such culture substrate surfaces can be created by using the living radical polymerization. Specifically, an easy and precise designing of cell substrate surfaces is possible under the Reversible Addition-Fragmentation Chain Transfer Radical Polymerization, and a continued reaction on the terminal of the molecular chain facilitates introduction of functional groups, so such polymerization is extremely advantageous for cell culturing.

EXAMPLES

The present invention is described in more detail below based on the Examples, without being limited by those Examples.

[Example 1]

A glass substrate was placed in a separable flask, then 500 $\mu$L of toluene solution comprising 2.5 $\mu$L of 3-aminopropyltriethoxysilane (APTES) was added to the flask and reacted at 150˚ C under a nitrogen atmosphere for 20 hours to obtain a glass substrate with an amino group introduced therein (APTES substrate). V-501, which is a polymerization initiator, was immobilized on the obtained APTES substrate to produce an initiator-immobilized substrate (V-501 substrate). Since the polymerization initiator V-501 contains carbonic acid, the immobilization of V-501 to the substrate was performed by immersing the APSES substrate in a mixed solution of V-501 (5.25 g) and 9.25 g of a concentrate, 1-(ethoxycarbonyl)-2-ethoxy-1,2-dihydroquinoline (EEDQ), and subjecting the substrate to a condensation reaction (25˚C, 20 hours). Then, the V-501 substrate was immersed in 1,4-dioxane containing a RAFT agent (0.25 mM), NIPAAm (1 M) and subjected to a polymerization reaction (70˚C, 20 hours).

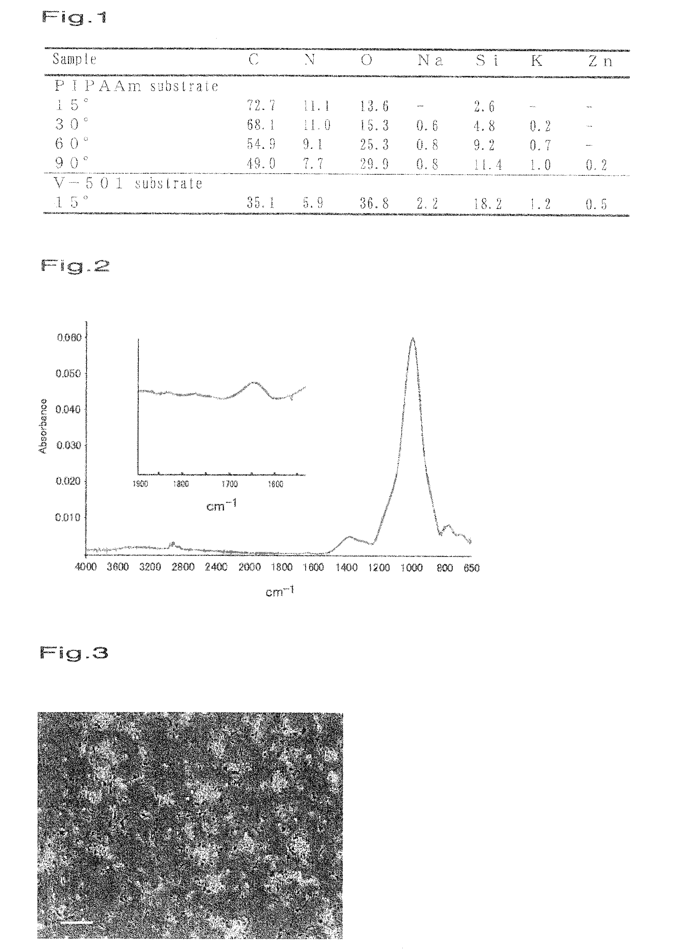

Free polymers are produced in the solution when PIPAAm is grafted to the substrate surface. The polymers produced in the solution were precipitated in diethyl ether and purified. The molecular weight of the collected free PIPAAm, measured by the gel permeation chromatography (GPC), was 105,620. Meanwhile, an X-ray photoelectron spectroscopy made clear that polymer was grafted to the V-501 substrate surface (Fig. 1). Further, the amount of grafted PIPAAm on the substrate was calculated from the FT-IR measurement result. The amount of grafted PIPAAm on the substrate was calculated by normalizing the peak derived from the carbonyl group of PIPAAm detected with dependence on the graft amount (around 1650 cm$^{-1}$) by the peak intensity derived from the glass substrate (1000 cm$^{-1}$) and by using the calibration curve based on the PIPAAm solution; the result was 0.65 $\mu$g/cm$^2$. The graft density was calculated from the molecular weight of the free PIPAAm and the amount of grafted PIPAAm, obtained by the above method. The graft density calculated using the following formula was 0.036 molecular chain/nm$^2$.

$$[\text{Graft density (molecular chain/nm}^2) = \text{PIPAAm graft amount (g/nm}^2)/\text{PIPAAm molecular weight} \times \text{Avogadro's number}]$$

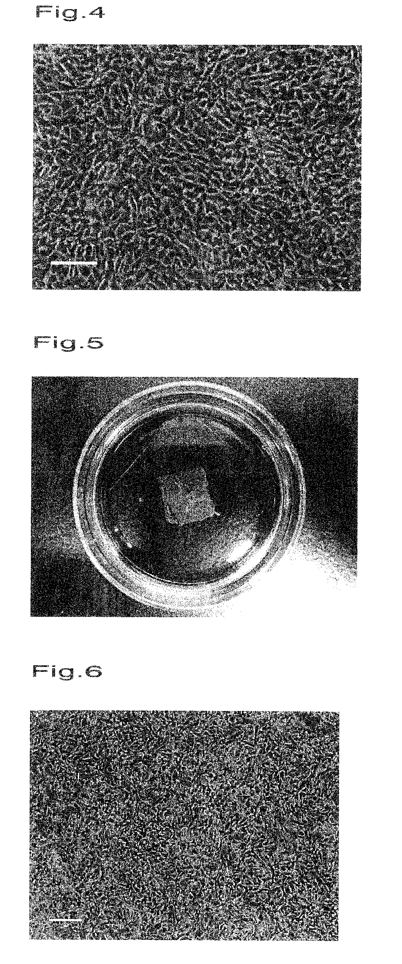

Cell adhesion was observed by microscopy as a result of disseminating carotid artery vascular endothelial cells of a cow ($1\times10^5$ cells/cm$^2$) on the obtained PIPAAm graft substrate (Fig. 3). However, the cell adhesion was relatively low, and 5 days passed before the cells reached confluence (Fig. 4). In contrast, the desorption rate was relatively high, and the cell sheet was completely detached from the surface after 30 minutes of low temperature processing (20˚C, 5% CO$_2$).

[Example 2]

In order to adjust the density of the amino group introduced onto the glass substrate, an APTES solution mixed with hexyltriethoxysilane (HTES), which is a silane agent containing an alkyl chain, was prepared to perform a silane coupling reaction. Specifically, 500 $\mu$L of toluene solution containing a mixture of (APTES 1.25 $\mu$L and HTES of the same mole as the APTES was poured into the separable flask with a glass substrate placed therein, then the solution was reacted at 150˚C for 20 hours (APTES/HTES substrate). An APTES/HTES substrate was immersed into a mixed solution of a polymerization initiator V-501 (5.25 g) and a concentrate EEDQ (9.25 g), then condensation reaction (25˚C, 20 hours) was induced to immobilize V-501 on the substrate. The V-501 substrate was immersed in 1,4-dioxane containing a RAFT agent (0.25 mM) and NIPAAm (1 M) to perform a polymerization reaction (70˚C, 20 hours).

The molecular weight of the free PIPAAm purified by precipitation in diethyl ether was measured by GPC to be 105,620. Further, the amount of grafted PIPAAm on the substrate was calculated from the FT-IR measurement result to be 0.43 $\mu$g/cm$^2$, The graft density was calculated from the molecular weight of the free PIPAAm and the amount of grafted PIPAAm, obtained by the above method. The graft density calculated using the following formula was 0.024 molecular chain/nm$^2$.

$$[\text{Graft density (molecular chain/nm}^2) = \text{PIPAAm graft amount (g/nm}^2)/\text{PIPAAm molecular weight} \times \text{Avogadro's number}]$$

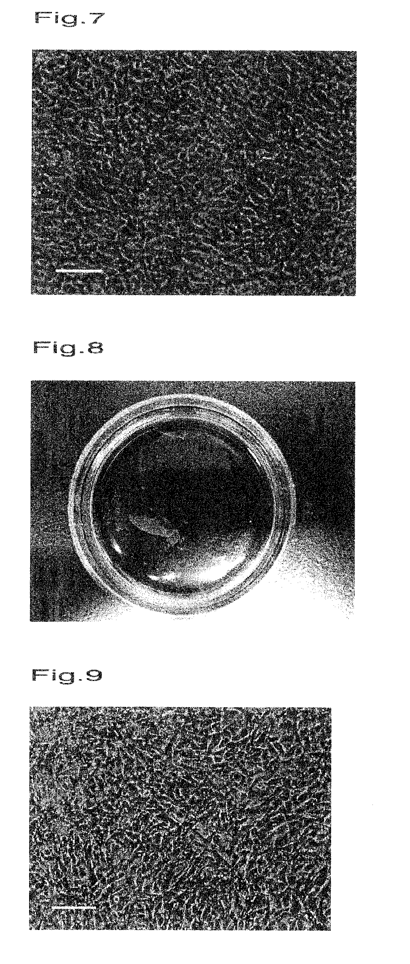

Cell adhesion was observed by microscopy as a result of disseminating carotid artery vascular endothelial cells of a cow ($1 \times 10^5$ cells/cm$^2$) on the obtained PIPAAm graft substrate. The cell adhesion tends to increase with decrease in density. Specifically, the number of surface-attached cells 6 hours after dissemination was clearly higher than that of Example 1 (Fig. 6). Further, cells were confluent 2 days after dissemination. This result shows that cell adhesion increased by decrease in the graft density (Fig. 7). The detachment of a cell sheet was observed after 30 minutes of low temperature processing (Fig. 8), so it was shown that the cell sheet can be collected at a density of 0.024 molecular chain/nm$^2$ or higher.

[Example 3]

A glass substrate was placed in a separable flask, then 500 $\mu$L of toluene solution comprising 2.5 $\mu$L of APTES was added to the flask and reacted at 150˚C under a nitrogen atmosphere for 20 hours to obtain a glass substrate with an amino group introduced therein (APTES substrate). V-501, which is a polymerization initiator, was immobilized on the obtained APTES substrate to produce an initiator-immobilized substrate (V-501 substrate). The immobilization of V-501 to the substrate was performed by immersing the APTES substrate in a mixed solution of V-501 (5.25 g) and 9.25 g of a concentrate EEDQ and subjecting the substrate to a condensation reaction (25˚C, 20 hours). Then, the V-501 substrate was immersed in 1,4-dioxane containing a RAFT agent (1 mM), NIPAAm (1 M) and subjected to a polymerization reaction (70˚C, 20 hours).

The molecular weight of the free PIPAAm purified by precipitation in diethyl ether was measured by GPC to be 68,284. Further, the amount of grafted PIPAAm was calculated from the FT-IR measurement result to be 0.41 $\mu$g/cm$^2$. The graft density was calculated from the molecular weight of the free PIPAAm and the amount of grafted PIPAAm, obtained by the above method. The graft density calculated using the following formula was 0.036 molecular chain/nm$^2$.

$$[\text{Graft density (molecular chain/nm}^2) = \text{PIPAAm graft amount (g/nm}^2)/\text{PIPAAm molecular weight} \times \text{Avogadro's number}]$$

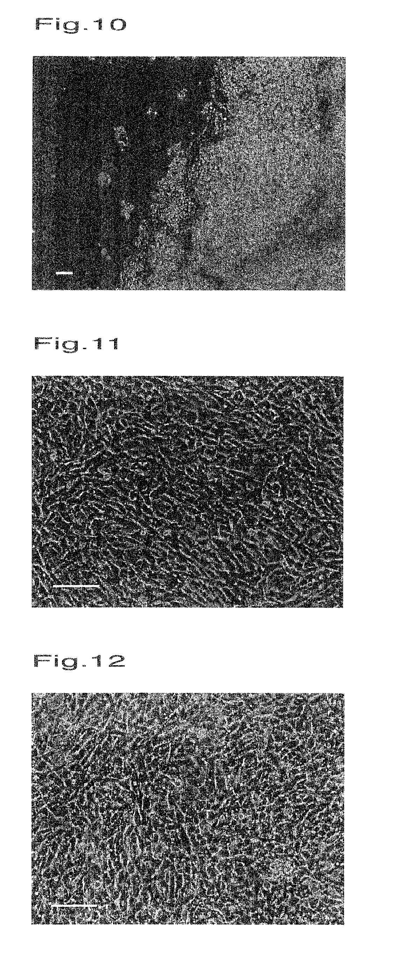

Cell adhesion was observed by microscopy as a result of disseminating carotid artery vascular endothelial cells of a cow ($1 \times 10^5$ cells/cm$^2$) on the obtained PIPAAm graft substrate (Fig. 9). To collect a cell sheet, cells, which had reached confluence 2 days after dissemination under a common culture condition (37˚C, 5%CO$_2$), were subjected to low temperature processing (20˚C, 5%CO$_2$). Consequently, a cell sheet was successfully collected after 1 hour of low temperature processing (Fig. 10).

[Comparative Example 1]

In order to adjust the density of the amino group introduced onto the glass substrate, an APTES solution mixed with HTES, which is a silane agent containing an alkyl chain, was prepared to perform a silane coupling reaction. Specifically, 500 $\mu$L of toluene solution containing a mixture of APTES 1.25 $\mu$L and HTES of the same mole as the APTES was poured into the separable flask with a glass substrate placed therein, then the solution was reacted at 150˚C for 20 hours. An APTES/HTES substrate was immersed into a mixed solution of a polymerization initiator V-501 (5.25 g) and a concentrate EEDQ (9.25 g), then condensation reaction (25˚C, 20 hours) was induced to immobilize V-501 on the substrate. The V-501 substrate was immersed in 1,4-dioxane containing a RAFT agent (1 mM) and NIPAAm (1 M) to perform a polymerization reaction (70˚C, 20 hours).

The molecular weight of the free PIPAAm purified by precipitation in diethyl ether was measured by GPC to be 68,284. Further, the amount of grafted PIPAAm on the substrate was calculated from the FT-IR measurement result to be 0.32 $\mu$g/nm$^2$. The graft density was calculated from the molecular weight of the free PIPAAm and the amount of grafted PIPAAm, obtained by the above method. The graft density calculated using the following formula was 0.027 molecular chain/nm$^2$.

$$[\text{Graft density (molecular chain/nm}^2) = \text{PIPAAm graft amount (g/nm}^2)/\text{PIPAAm molecular weight} \times \text{Avogadro's number}]$$

Cell adhesion was observed by microscopy as a result of disseminating carotid artery vascular endothelial cells of a cow ($1\times10^5$ cells/cm$^2$) on the obtained PIPAAm graft substrate. The cell adhesion was relatively high as in Example 3, and the cells reached confluence 2 days after dissemination (Fig. 11). However, the cell sheet did not peel off by low temperature processing (20°C, 5%CO$_2$) (Fig. 12). Since cell sheets peeled off from substrates of grafted PIPAAms of the same chain length, polymerized under the same conditions (Example 3), it is understood that the density must be higher than 0.027 molecular chain/nm$^2$ to collect a cell sheet from the PIPAAm graft substrate when the molecular weight of PIPAAm is 68,284. The cell sheet peeled off from a surface having a density of 0.024 molecular chain/nm$^2$ when the molecular weight was 105,620 (Example 2). This result suggested that both the molecular chain length and density contribute to the temperature-responsiveness of a surface.

[Comparative Example 2]

A glass substrate was placed in a separable flask, then 500 $\mu$L of toluene solution comprising 2.5 $\mu$L of APTES was added to the flask and reacted at 150°C under a nitrogen atmosphere for 20 hours to obtain a glass substrate with an amino group introduced therein (APTES substrate). The immobilization of V-501 to the substrate was performed by immersing the APTES substrate in a mixed solution of V-501 (5.25 g) and 9.25 g of a concentrate EEDQ and subjecting the substrate to a condensation reaction (25°C, 20 hours). Then, the V-501 substrate was immersed in 1,4-dioxane containing NIPAAm (2 M) and subjected to a polymerization reaction (70°C, 20 hours). The polymerization reaction progressed without the co-existence of a RAFT agent, and PIPAAm was grafted onto the substrate surface without being subjected to RAFT polymerization.

The molecular weight of the free PIPAAm purified by precipitation in diethyl ether was measured by GPC to be 333,060. Further, the amount of grafted PIPAAm on the substrate was calculated from the FT-IR measurement result to be 3.30 $\mu$g/nm$^2$. The graft density was calculated from the molecular weight of the free PIPAAm and the amount of grafted PIPAAm, obtained by the above method. The graft density calculated using the following formula was 0.059 molecular chain/nm$^2$.

$$[\text{Graft density (molecular chain/nm}^2) = \text{PIPAAm graft amount (g/nm}^2)/\text{PIPAAm molecular weight} \times \text{Avogadro's number}]$$

No cell adhesion was observed by microscopy as a result of disseminating carotid artery vascular endothelial cells of a cow ($1\times10^5$ cells/cm$^2$) on the obtained PIPAAm graft substrate. PIPAAm grafted in a solution not containing a RAFT agent has an extremely long chain length and many grafts. It is considered that the PIPAAm brush surface shows high hydrophilicity and acts as an adhesion resistant surface against cells.

[Example 4]

A glass substrate was placed in a separable flask, then 500 $\mu$L of toluene solution comprising 2.5 $\mu$L of 3-aminopropyltriethoxysilane (APTES) was added to the flask and reacted at 150°C under a nitrogen atmosphere for 20 hours to obtain a glass substrate with an amino group introduced therein (APTES substrate). V-501, which is a polymerization initiator, was immobilized on the obtained APTES substrate to produce an initiator-immobilized substrate (V-501 substrate). Since the polymerization initiator V-501 contains carbonic acid, the immobilization of V-501 to the substrate (V-501 substrate) was performed by immersing the APTES substrate in a mixed solution of V-501 (5.25 g) and 9.25 g of a concentrate, 1-(ethoxycarbonyl)-2-ethoxy-1,2Wdihydroquinoline (EEDQ), and subjecting the substrate to a condensation reaction (25°C, 20 hours). Then, the V-501 substrate was immersed in 1,4-dioxane containing a RAFT agent (0.5 mM), NIPAAm (1 M) and subjected to a polymerization reaction (70°C, 20 hours).

Carboxyl groups were introduced on the PIPAAm brush terminals by immersing the obtained PIPAAm graft substrate in a PBS solution containing 3-maleimidopropionic acid (30 mM) and 2-aminoethanol (10 mM). The substrate was further immersed in a PBS solution containing 1-ethyl-3-(3-dimethylaminopropyl)carbodiimide (50 mg/mL) and N-hydroxysuccinimide (NHS) (10 mg/mL), stirred at 20°C for 1 hour to produce a brush having NHS esters on its terminals. The NHS-modified PIPAAm graft substrate was immersed in a GRGDS solution (100 $\mu$M) to produce an RGD-modified PIPAAm brush surface.

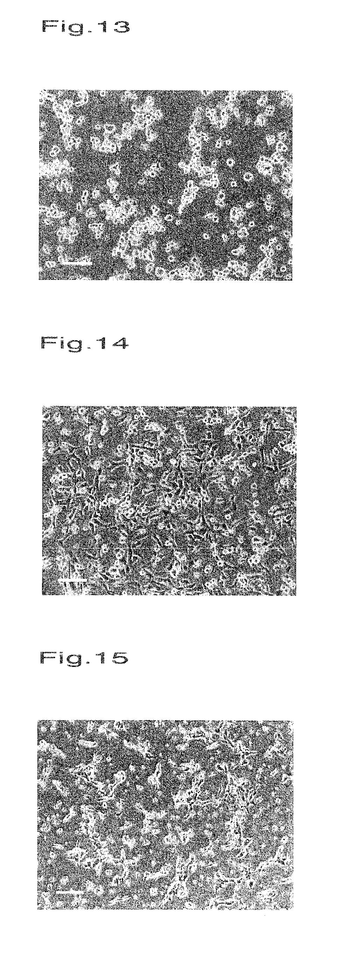

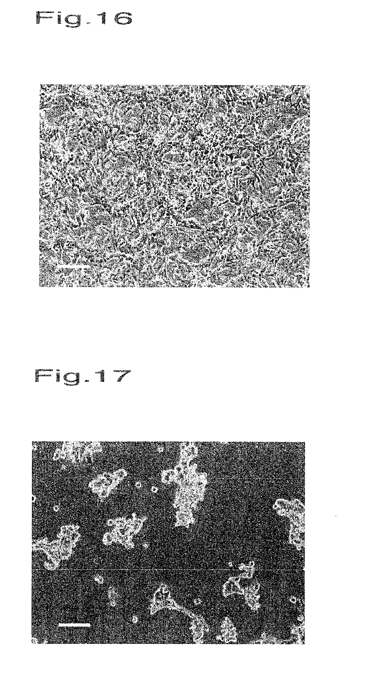

Human renal proximal tubule epithelial cells (RPTEC) have extremely low adherence to glass substrates, and culturing the cells to confluence was difficult, even if they were disseminated at a relatively high dissemination density. Hence, RPTEC was disseminated on the surface of an RGD-modified PIPAAm brush surface to assess the effects of RGD introduced on the terminal. When RPTEC was disseminated on the surface of the obtained PIPAAm brush ($1 \times 10^5$ cells/cm$^2$), the cells that were disseminated on the brush surface of PIPAAm having unmodified terminals were not extended after 3 hours (Fig. 13). In contrast, the cells that were disseminated on the surface of an RGD-modified PIPAAm brush were mostly attached to the substrate and extending 3 hours after dissemination, and exhibited improved adhesion due to RGD modification (Fig. 14). The result suggests an induction of cell adhesion via specific interactions of RGD peptides and cells. The adhesion of cells to the surface of the non-modified PIPAAm brush was almost non-existent after 24 hours of culture (Fig. 15), and the cells remained non-confluent after an additional few days of culture. in contrast, the cells attached to the surface of an RGD-modified PIPAAm brush were mostly confluent 24 hours after dissemination (Fig. 16). Further, the adhered cells desorbed quickly from the surface of the RGD-modified PIPAAm brush as the temperature changed (20˚C) (Fig. 17). The above result shows that the cell adhesion to the surface of a PIPAAm brush improves by RGD modification. Further, the RGD-modified surface can induce efficient detachment of cultured cells by cooling alone, so such surface functions sufficiently as a temperature-responsive surface.

INDUSTRIAL APPLICABILITY

The use of a temperature-responsive cell culture substrate described in the present invention enables efficient culture of cells obtained from different tissues. The use of such culture method enables cell sheets to be peeled off efficiently and without damage, by temperature change alone.

**Claims**

1. A temperature-responsive cell culture substrate composed of a non-crosslinked temperature-responsive polymer having a molecular weight of 10,000 to 150,000 and immobilized on a substrate surface at a density of 0.02 to 0.3 molecular chain/nm$^2$

2. The temperature-responsive cell culture substrate according to claim 1, wherein the temperature-responsive polymer immobilized on the substrate surface has hydration that changes in a temperature range of 0˚C to 80˚C.

3. The temperature-responsive cell culture substrate according to either claim 1 or 2, wherein the temperature-responsive polymer consists of one of a poly-N-substituted acrylamide derivative, a poly-N-substituted methacrylamide derivative, a polyacrylate derivative, a polymethacrylate derivative or a copolymer of two or more of a poly-N-substituted acrylamide derivative, a poly-N-substituted methacrylamide derivative, a polyacrylate derivative, a polymethacrylate derivative.

4. The temperature-responsive cell culture substrate according to any one of claims 1 to 3, having a functional group on a terminal of an immobilized temperature-responsive polymer chain.

5. The temperature-responsive cell culture substrate according to claim 4, wherein the functional group is one of a dithioester group, a hydroxyl group, a carboxyl group, and an amino group or a mixture of two or more of a dithioester group, a hydroxyl group, a carboxyl group, and an amino group.

6. A method for manufacturing the temperature-responsive cell culture substrate according to any one of claims 1 to 5, comprising immobilizing the temperature-responsive polymer on the substrate surface by a living radical polymerization.

7. The method according to claim 6, wherein the polymerization is Reversible Addition-Fragmentation Chain Transfer Radical Polymerization.

8. The method according to either claim 6 or 7, wherein an azo polymerization initiator immobilized on the substrate surface is used for the polymerization.

9. The method according to any one of claims 6 to 8, comprising:

converting a dithioester group on a terminal of an immobilized temperature-responsive polymer chain to a thiol group; and
further converting the thiol group to a hydroxyl group, a carboxyl group, or an amino group.

Fig.1

| Sample | C | N | O | Na | Si | K | Zn |
|---|---|---|---|---|---|---|---|
| PIPAAm substrate | | | | | | | |
| 15° | 72.7 | 11.1 | 13.6 | – | 2.6 | – | – |
| 30° | 68.1 | 11.0 | 15.3 | 0.6 | 4.8 | 0.2 | – |
| 60° | 54.9 | 9.1 | 25.3 | 0.8 | 9.2 | 0.7 | – |
| 90° | 49.0 | 7.7 | 29.9 | 0.8 | 11.4 | 1.0 | 0.2 |
| V-501 substrate | | | | | | | |
| 15° | 35.1 | 5.9 | 36.8 | 2.2 | 18.2 | 1.2 | 0.5 |

Fig.2

Fig.3

Fig.4

Fig.5

Fig.6

Fig.7

Fig.8

Fig.9

Fig.10

Fig.11

Fig.12

Fig.13

Fig.14

Fig.15

Fig.16

Fig.17

<div style="text-align:center">**INTERNATIONAL SEARCH REPORT**</div>

| | International application No. |
|---|---|
| | PCT/JP2010/064627 |

| A. CLASSIFICATION OF SUBJECT MATTER |
|---|
| *C12M3/00*(2006.01)i, *C12N5/071*(2010.01)n |

According to International Patent Classification (IPC) or to both national classification and IPC

| B. FIELDS SEARCHED |
|---|
| Minimum documentation searched (classification system followed by classification symbols) |
| C12M3/00, C12N5/071 |

Documentation searched other than minimum documentation to the extent that such documents are included in the fields searched

| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
JSTPlus/JMEDPlus/JST7580(JDreamII)

| C. DOCUMENTS CONSIDERED TO BE RELEVANT |
|---|

| Category* | Citation of document, with indication, where appropriate, of the relevant passages | Relevant to claim No. |
|---|---|---|
| X/Y | JP 2007-69193 A  (Mitsuo OKANO),<br>22 March 2007 (22.03.2007),<br>claims; paragraphs [0033] to [0035], [0038]<br>(Family: none) | 1-3,6/4,5,<br>7-9 |
| Y | NAKAYAMA M et al, Polymer terminal group effects on properties of thermoresponsive polymeric micelles with controlled outer-shell chain lengths, Biomacromolecules, 2005, vol.6, p.2320-2327 | 4,5,7-9 |
| Y | JP 2002-60671 A  (Mitsubishi Chemical Corp.),<br>26 February 2002 (26.02.2002),<br>abstract<br>(Family: none) | 4,5,9 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 18 November, 2010 (18.11.10) | 30 November, 2010 (30.11.10) |

| Name and mailing address of the ISA/ | Authorized officer |
|---|---|
| Japanese Patent Office | |
| Facsimile No. | Telephone No. |

**INTERNATIONAL SEARCH REPORT**

| | International application No. |
|---|---|
| | PCT/JP2010/064627 |

C (Continuation).   DOCUMENTS CONSIDERED TO BE RELEVANT

| Category* | Citation of document, with indication, where appropriate, of the relevant passages | Relevant to claim No. |
|---|---|---|
| Y | WO 01/68799 A1  (Mitsuo OKANO),<br>20 September 2001 (20.09.2001),<br>examples 1, 2<br>& US 2003/0036196 A1    & EP 1264877 A1 | 1-9 |
| A | JP 2004-331776 A  (Organo Corp.),<br>25 November 2004 (25.11.2004),<br>& US 2007/163332 A1    & EP 1630193 A1 | 1-9 |
| E,A | JP 2010-98973 A  (Konica Minolta Holdings, Inc.),<br>06 May 2010 (06.05.2010),<br>(Family: none) | 1-9 |
| T | Hironobu TAKAHASHI et al., "Ondo Otosei Ko Bunshi Brush Hyomen ni Okeru Saibo no Secchaku·Dacchaku Kyodo no Kisoteki Kento", Polymer Preprints, Japan, 01 September 2009 (01.09.2009), vol.58, no.2, pages 5030 to 5031 | 1-9 |
| T | Hironobu TAKAHASHI et al., "Saibo Sheet Sakusei o Mokuteki to shita Ondo Otosei Polymer Brush Hyomen no Bunshi Sekkei", The Annual Meeting of the Japanese Society for Biomaterials Yokoshu, 16 November 2009 (16.11.2009), vol.31, page 201 | 1-9 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H02211865 B
- JP H05192138 B
- JP 2008220354 A
- JP H08103653 B
- JP H07135957 B

**Non-patent literature cited in the description**

- *Biotechnology and Bioengineering,* 2008, vol. 101 (6), 1321-1331
- *Biomaterials,* 2008, vol. 29, 2073-2081
- *Aust. J. Chem.,* 2005, vol. 58, 379-410
- *Biopolymers,* 2005, vol. 6, 2320-2327
- *Macromolecules,* 2005, vol. 38, 5937-5943